# EUROPEAN PATENT SPECIFICATION

(11) **EP 1 976 391 B1**
(45) Date of publication and mention of the grant of the patent: **20.10.2010**
(21) Application number: 05822906.3
(22) Date of filing: 30.12.2005
(51) Int. Cl.: A23G 4/06

(54) **CHEWING GUM COMPOSITION CONTAINING CHOKEBERRY**
KAUGUMMIZUSAMMENSETZUNG MIT ARONIA
COMPOSITION DE GOMME A MACHER CONTENANT DE L'ARONIA

(43) Date of publication of application: 08.10.2008
(73) Proprietor: Cadbury Holdings Limited, Uxbridge, Middlesex UB8 1DH (GB)
(72) Inventor: PORSGAARD, Tania Kjølhede, 8220 Brabrand (DK)
(74) Representative: Plougmann & Vingtoft A/S
(86) International application number: PCT/DK2005/000840
(87) International publication number: WO 2007/076857

(56) References cited:
- EP-A- 1 208 755
- EP-A- 1 607 006
- WO-A-01/14875
- US-A1- 2005 013 880

## Description

### FIELD OF THE INVENTION

The present invention relates to a chewing gum composition containing chokeberry. The invention furthermore relates to methods of preparing the chewing gum composition, and to uses of the chewing gum composition.

### BACKGROUND

Chokeberry is an interesting berry from a nutritional and medical point of view. It contains large quantities of antioxidants, and it is believed to have health benefits such as a prophylactic effect against cancer, heart diseases and Alzheimer's disease.

One of the major reasons why chokeberry has not gained common popularity, like for example cranberry, is its taste. Chokeberry often is intensely bitter and therefore difficult to use in food products and medicinal products for oral administration.

EP 1 208 755 discloses black currant anthocyanin-containing compositions for foods comprising 1 to 25% by weight of black currant anthocyanin on the basis of solid matters and a process for producing a black currant anthocyanin-containing composition for foods.

WO 01/14875 discloses a pH sensitive chewing gum compositions, said chewing gum compositions may comprise anthocyanin derived from red cabbage.
US 2005/013880 discloses pharmaceutical compositions, e.g. chewing gum, and methods for inhibiting the growth and cell cycle progression in carcinoma cells comprising the step of contacting the cells with an anthocyanin rich extract (ARE), preferably derived from chokeberry in an amount effective to inhibit the growth and cell cycle progression of the carcinoma cells without effecting the growth and cell cycle progression on normal cells.

EP 1 607 006 discloses food composition comprising elderberry and/or blackcurrant, and optionally in addition black chokeberry, wherein the food composition has a polyphenol content of 300 mg/kg or more, calculated as gallic acid equivalents, wherein the consumption of the food composition by a human subject is effective in reducing the cardiovascular risk of that human subject.

Therefore there is a general need for chokeberry products with a consumer acceptable taste.

### SUMMARY OF THE INVENTION

An object of the present invention is to provide a food product comprising chokeberry and having a consumer acceptable taste.

Other objects of the invention will become apparent when reading the description and the examples.

An aspect of the present invention relates to a chewing gum composition comprising a mixture comprising at least one chewing gum ingredient, gum base, and chokeberry, the mixture comprising gum base in an amount of 10-70% by weight.

The inventor has discovered that the chewing gum composition according to the invention has the unique effect that is reduces the inherent bitterness of chokeberry. Without being bound by theory, it is believed that this effect may be based on a controlled, delayed release of chokeberry, which is mixed with gum base. Instead of an initial burst of chokeberry, which normally takes place when chokeberry is enjoyed as a juice or as raw fruit, chokeberry is slowly released while the chewing gum composition is chewed. The controlled, delayed release effect is documented in Examples 2 and the taste-masking effect is documented in Example 3.

The inventor furthermore expects that the chewing gum composition can be used for removal of plaque and/or biofilm from the teeth, thus the chewing gum composition may be used for cosmetic removal of plaque and/or biofilm.

Chokeberry is believed to inhibit the colonization of streptococci in the initial phase of biofilm formation hence slow the development of dental plaque. Particularly, the high molecular weight of proanthocyanins from chokeberry is believed to inhibit the biofilm formation of oral Streptococcus.

Another aspect of the present invention related to a method of producing the chewing gum composition comprising chokeberry.

Yet another aspect of the invention relates to uses of chokeberry and of the chewing gum composition comprising chokeberry.

### BRIEF DESCRIPTION OF THE FIGURE

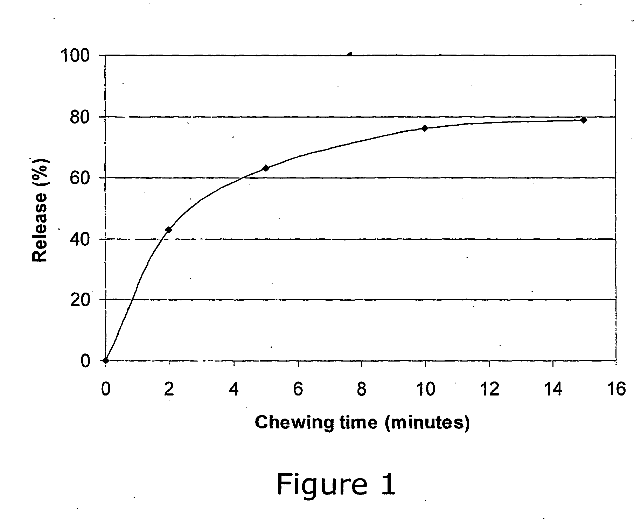

Figure 1 is a plot of the result of the release experiment performed in Example 2.

### DETAILED DESCRIPTION OF THE INVENTION

An aspect of the present invention relates to a chewing gum composition comprising a mixture comprising at least one chewing gum ingredient, gum base, and chokeberry, the mixture comprising gum base in an amount of 10-70% by weight.

In an embodiment of the invention the mixture comprises chokeberry in an amount in the range of 0.1-30% by weight. Preferably, the mixture comprises chokeberry in an amount in the range of 0.5-25% by weight such as 1-20% by weight.

In another embodiment of the invention the mixture comprises chokeberry in an amount in the range of 15-60% by weight, such as e.g. in the range of 20-50% by weight.

Thus, in an embodiment of the invention the mixture comprises gum base in an amount in the range of 10-70% by weight, preferably in an amount in the range of 20-60% by weight, and even more preferred in an amount in the range 30-50% by weight such as 35-45% by weight.

The inventor has additionally found that, besides a very intense colouring effect, chokeberry also functions as a pH indicator when incorporated into the mixture comprising gum base. At an acidic pH chokeberry is intensely red/purple, and above pH 5.5 it is blue. This makes it possible to measure the pH in the mouth cavity by chewing the present chewing gum composition.

In a preferred embodiment of the invention, the chewing gum composition furthermore comprises a coating.

Typically the chewing gum composition comprises the coating in an amount in the range of 1-80% by weight, such as in an amount in the range of 10-50%, or 15-45% by weight. Preferably, the chewing gum composition comprises the coating in an amount in the range of 20-40% by weight.

In an embodiment of the invention, the coating also comprises chokeberry. The coating may e.g. comprise chokeberry an amount in the range of 0.1-30% by weight of the coating, such as in the range of 0.5-20% by weight. Preferably, the coating comprises chokeberry an amount in the range of 1-10% by weight of the coating.

The coating may be a hard coating, which term is used in the conventional meaning of that term including sugar coatings and sugar-free (or sugarless) coatings and combinations thereof. The objects of hard coating are to obtain a sweet, crunchy layer, which is appreciated by the consumer, and to protect the mixture for various reasons. In a typical process of providing the mixture with a protective sugar coating the gum centers are successively treated in suitable coating equipment with aqueous solutions of crystallizable sugar such as sucrose or dextrose, which, depending on the stage of coating reached, may contain other functional ingredients, e.g. fillers, colours, etc. In the present context, the sugar coating may contain further functional or active compounds including flavour compounds, pharmaceutically active compounds and/or polymer degrading substances.

In the production of chewing gum compositions it may, however, be preferred to replace the cariogenic sugar compounds in the coating by other, preferably crystallizable, sweetening compounds that do not have a cariogenic effect. In the art such coating is generally referred to as sugarless or sugar-free coatings. Presently preferred non-cariogenic hard coating substances include polyols, e.g. sorbitol, maltitol, mannitol, xylitol, erythritol, lactitol, isomalt and tagatose which are obtained by industrial methods by hydrogenation of D-glucose, maltose, fructose or levulose, xylose, erythrose, lactose, isomaltulose and D-galactose, respectively.

In a typical hard coating process, a syrup containing crystallizable sugar and/or polyol is applied onto the mixture and the water it contains is evaporated off by blowing with warm, dry air. This cycle may be repeated several times, typically 10 to 80 times, in order to reach the swelling required. The term "swelling" refers to the increase in weight of the products, as considered at the end of the coating operation by comparison with the beginning, and in relation to the final weight of the coated chewing gum composition.

Alternatively, the coating may be a soft coating. Such a soft coating is applied using conventional methods and may advantageously consist of a mixture of a sugar or any of the above non-cariogenic, sugar-less sweetening compounds and a starch hydrolysate.

The coating may be a film coating. The film coating may be obtained by subjecting the mixture to a film coating process and which therefore comprises one or more film-forming polymeric agents and optionally one or more auxiliary compounds, e.g. plasticizers, pigments and opacifiers. A film coating is a thin polymer-based coating applied to a mixture of any of the above forms. The thickness of such a film coating is usually between 20 and 100 µm. Generally, the film coating is obtained by passing the mixture through a spray zone with atomized droplets of the coating materials in a suitable aqueous or organic solvent vehicle, after which the material adhering to the mixture is dried before the next portion of coating is received. This cycle is repeated until the coating is complete.

In the present context, suitable film-coating polymers include edible cellulose derivatives such as cellulose ethers including methylcellulose (MC), hydroxyethyl cellulose (HEC), hydroxypropyl cellulose (HPC) and hydroxypropyl methylcellulose (HPMC). Other useful film-coating agents are acrylic polymers and copolymers, e.g. methylacrylate aminoester copolymer or mixtures of cellulose derivatives and acrylic polymers. A particular group of film-coating polymers also referred to, as functional polymers are polymers that, in addition to its film-forming characteristics, confer a modified release performance with respect to active components of the chewing gum formulation. Such release modifying polymers include methylacrylate ester copolymers, ethylcellulose (EC) and enteric polymers designed to resist the acidic stomach environment, yet dissolve readily in the duodenum. The latter group of polymers includes: cellulose acetate phtalate (CAP), polyvinyl acetate phtalate (PVAP), shellac, metacrylic acid copolymers, cellulose acetate trimellitate (CAT) and HPMC. It will be appreciated that the outer film coating according to the present invention may comprise any combination of the above film-coating polymers.

In other embodiments of the invention, the film-coating layer of the chewing gum composition comprise a plasticizing agent having the capacity to alter the physical properties of a polymer to render it more useful in performing its function as a film forming material. In general, the effect of plasticizers will be to make the polymer softer and more pliable as the plasticizer molecules interpose themselves between the individual polymer strands thus breaking down polymer-polymer interactions. Most plasticizers used in film coating are either amorphous or have very little crystallinity.

In the present context, suitable plasticizers include polyols such as glycerol, propylene glycol, polyethylene glycol, e.g. the 200-6000 grades hereof, organic esters such as phtalate esters, dibutyl sebacate, citrate esters and thiacetin, oils/glycerides including castor oil, acetylated monoglycerides and fractionated coconut oil.

The choice of film-forming polymer (s) and plasticizing agent (s) for the film coating of the mixture is made with due consideration for achieving the best possible barrier properties of the coating in respect of dissolution and diffusion across the film of moisture and gasses.

The film coating of the mixture may also contain one or more colorants or opacifiers. In addition to providing a desired colour hue, such agents may contribute to protecting the compressed gum base against pre-chewing reactions, in particular by forming a barrier against moisture and gasses. Suitable colorants/opacifiers include organic dyes and their lakes, inorganic colouring agents, e.g. titanium oxide and natural colours such as e.g. beta-carotene.

Additionally, film coatings may contain one or several auxiliary substances such as flavours and waxes or saccharide compounds such as polydextrose, dextrins including maltodextrin, lactose, modified starch, a protein such as gelatine or zein, a vegetable gum and any combination thereof.

The coating, in general, typically comprises one or more layers. For example the number of layers of the coating may be in the range of 1-100 layers, such as 3-75 layers, 10-60 layers, and 20-40 layers.

The coating comprises for example comprise a wax layer. In an embodiment of the invention, the outermost layer of the coating is a wax layer.

The chewing gum composition normally comprises the mixture in an amount in the range of 10-100% by weight of the chewing gum composition. When the chewing gum composition is an uncoated chewing gum composition, the mixture may constitute 100% or nearly 100% by weight of the chewing gum composition. In the case of center filled and/or coated chewing gum compositions, the chewing gum composition normally comprises the mixture in an amount in the range of 10-95% by weight, preferably in an amount in the range of 40-85% by weight, and even more preferred in an amount in the range 60-80% by weight.

A center filled chewing gum composition may furthermore comprise chokeberry in the center-fill and/or in a coating.

In the present context the term "chokeberry" relates to berries of plants of the family Rosaceae and genus Aronia. There are three species within the Aronia-genus, that is, Aronia Melanocarpa (Black chokeberry), Aronia arbutifolia (red chokeberry), and Aronia prunifolia (purple chokeberry). In some embodiments, the chokeberry preferably is black chokeberry.

The term "chokeberry" may relate to chokeberry in different forms, e.g. raw chokeberry or processed chokeberry.

For example, chokeberry may be applied to the mixture in the form of fresh chokeberry, dried chokeberry, a chokeberry juice, or a chokeberry extract. In the case of chokeberry juice or a chokeberry extract, the chokeberry in the mixture will comprise the dry-matter of the juice or extract and possibly some residual water from the juice or residual solvent from the extract.

The juice may for example be an up-concentrated juice relative to the juice natural squeezed out of fresh chokeberry. The juice may e.g. be up-concentrated by evaporation of water.

Alternatively, or as a supplement, chokeberry may be in the form of a granulate when applied to the mixture.

In an embodiment of the invention, the granulate is prepared by drying, preferably freeze-drying, chokeberries and preparing the granulate from the dried or freeze-dried chokeberries, e.g. by grinding, milling, or slicing.

In another embodiment of the invention, the granulate is prepared by encapsulating chokeberry. The material to be encapsulated can e.g. be a chokeberry juice, a chokeberry extract, a powder of dried and/or freeze-dried chokeberries, or a powder of the dry-matter of a chokeberry juice or a chokeberry extract. Different methods of encapsulating include spray drying, spray cooling, film coating, coascervation, double emulsion method (extrusion technology) or prilling.

Encapsulation materials to be used for the above-mentioned encapsulation methods may e.g. include proteins, such as gelatine, wheat protein, soya protein, sodium caseinate, caseine, and/or zein; gums such as gum arabic; xanthan gum, locus bean gum; starches such as modified starch, hydrolyzed starches (maltodextrines); alginates; pectin; carregeenan; chitosan; waxes such as bees wax, candelilla wax, and/or carnauba wax; hydrogenated vegetable oils.

Other encapsulation materials which are useful for the above-mentioned encapsulation methods are polymers such as polymers polyvinyl alcohol, low molecular weight polyethylene; polyvinyl esters such as polyvinyl acetate, polyvinyl propionate; graft-copolymers of polyvinyl propionate and polyvinyl acetate, copolymers of vinyl acetate and ethylene, propylene, acrylic and methacrylic acid, crotonic acid, maleic acid and esters of unsaturated acids thereof.

In yet another embodiment, the granulate is prepared by removing the water from chokeberry juice, e.g. by drying or freeze-drying, to obtain the dry-matter of the juice. In this case the granulate would be a powder of the dry matter for chokeberry juice.

Thus, the chokeberry may be a powder or granulate of the dry-matter of a chokeberry juice or a chokeberry extract. Also, or alternatively, the chokeberry may be a powder or granulate of dried and/or freeze-dried chokeberries.

In a preferred embodiment of the invention the water content of the granulate is at most 5% by weight, preferably at most 1% by weight, and even more preferred at most 0.5% by weight.

As said, chokeberry may be encapsulated chokeberry. The encapsulated chokeberry may e.g. be an encapsulated chokeberry juice, it may be an encapsulated chokeberry extract, and it may be an encapsulated chokeberry granulate. For example, the chokeberry granulate to be encapsulated may be a powder of dried or freeze-dried chokeberries, it may be a powder of the dry-matter of a chokeberry juice or a chokeberry extract, or it may be a combination thereof.

Chokeberry, and particularly black chokeberry, contains considerable amount of anthocyanins. Anthocyanins are potent antioxidants and the chewing gum composition of the invention can therefore advantageously be used as a source of antioxidant.

In the present context the term "anthocyanin" is meant to encompass both single anthocyanin molecules as such as well as so-called proanthocyanins which are oligomers or polymers of anthocyanin molecules.

In an embodiment of the invention, chokeberry comprises at least 0.5% anthocyanin by weight, preferably at least 1% anthocyanin by weight, and even more preferred at least 5% anthocyanin by weight.

It may even be preferred that chokeberry comprises at least 10% anthocyanin by weight, such as at least 20% anthocyanin by weight, such as at least 30% anthocyanin by weight.

Highly concentrated forms of chokeberry, e.g. obtainable via up-concentrating chokeberry juice, is also envisioned and in this case chokeberry comprises at least 50% anthocyanin by weight, preferably at least 75% anthocyanin by weight, and even more preferred at least 90% anthocyanin by weight.

For example chokeberry may comprises anthocyanin in the range of 0.5-70% by weight, such as in the range of 5-60% by weight, preferably in the range of 10-50% by weight, and even more preferred in the range of 35-45% by weight.

In an embodiment of the invention the anthocyanin from chokeberry is present in the mixture of the chewing gum composition in an amount in the range of 0.01-20% by weight of the mixture, preferably in the range 0.01-10% by weight, such as in an amount in the range of 0.02-5% by weight. It is even more preferred that the anthocyanin from chokeberry is present in the mixture in an amount in the range of 0.03-3% by weight, such as e.g. in an amount in the range of 0.05-1% by weight.

The anthocyanin from chokeberry may for example be present in the mixture in an amount in the range of 0.4-0.6% by weight.

The inventor has discovered a surprising additional effect of the chewing gum composition, namely its ability to change colour after 3-5 minutes of chewing. Without being bound by theory it is believed that chokeberry inherently is acidic and slowly is titrated by the neutral saliva. The colour change, which is observed after prolonged chewing, is useful to signal to the consumer that now the chewing gum has been sufficiently chewing. This is particularly important and useful in the case of chewing gum compositions containing active ingredient such as pharmaceuticals and nutraceuticals. The active ingredient must normally be release in a certain dose to be effective, and the colour change of the chewing gum composition may signal that active ingredient now has been sufficiently released/"chewed out". In the present context this effect is called "chew out" indication.

The gum base of the mixture normally comprises an elastomer. Useful synthetic elastomers include, but are not limited to, synthetic elastomers listed in U.S. Food and Drug Administration, CFR, Title 21, Section 172,615, the Masticatory Substances, Synthetic, the contents of which are incorporated herein by reference for all purposes) such as polyisobutylene. e.g. having a gas pressure chromatography (GPC) average molecular weight in the range of about 10,000 to 1,000,000 including the range of 50,000 to 80,000, isobutylene-isoprene copolymer (butyl elastomer), styrene- butadiene copolymers e.g. having styrene-butadiene ratios of about 1:3 to 3:1, polyvinyl acetate (PVA), e.g. having a GPC average molecular weight in the range of 2,000 to 90,000 such as the range of 3,000 to 80,000 including the range of 30,000 to 50,000, where the higher molecular weight polyvinyl acetates are typically used in bubble gum base, polyisoprene, polyethylene, vinyl acetate-vinyl laurate copolymer e.g. having a vinyl laurate content of about 5 to 50% by weight such as 10 to 45% by weight of the copolymer, and combinations hereof.

It is possible to combine a synthetic elastomer having a high molecular weight and a synthetic elastomer having a low molecular weight elastomer in a gum base. Presently preferred combinations of synthetic elastomers include, but are not limited to, polyisobutylene and styrene-butadiene, polyisobutylene and polyisoprene, polyisobutylene and isobutylene-isoprene copolymer (butyl rubber) and a combination of polyisobutylene, styrene-butadiene copolymer and isobutylene isoprene copolymer, and all of the above individual synthetic polymers in admixture with polyvinyl acetate, vinyl acetate-vinyl laurate copolymers, respectively and mixtures thereof.

Typically, the gum base comprises at least one elastomer in an amount in the range of 3-80% by weight of the gum base, preferably in an amount in the range of 4-60% by weight of the gum base, and even more preferred in the range of 5-40% by weight of the gum base, such as in the range of 8-20% by weight of the gum base.

The elastomer may comprise a biodegradable elastomer. A biodegradable elastomer may e.g. be a polyester, e.g. obtained by polymerization of at least one cyclic ester.

The gum base may comprise one or more resins contributing to obtain the desired masticatory properties and acting as plasticizers for the elastomers of the gum base. In the present context, useful resins include, but are not limited to, natural rosin esters, often referred to as ester gums including as examples glycerol esters of partially hydrogenated rosins, glycerol esters of polymerised rosins, glycerol esters of partially dimerised rosins, glycerol esters of tally oil rosins, pentaerythritol esters of partially hydrogenated rosins, methyl esters of rosins, partially hydrogenated methyl esters of rosins and pentaerythritol esters of rosins. Other useful resinous compounds include synthetic resins such as terpene resins derived from alpha-pinene, beta-pinene, and/or d-limonene, natural terpene resins; and any suitable combinations of the foregoing. The choice of resins will vary depending on the specific application, and on the type of elastomer(s) being used.

Usually, the gum base comprises at least one resin in an amount in the range of 10-90% by weight of the gum base, preferably in the range of 20-80% by weight, even more preferred in the range of 30-70% by weight of the gum base, such as in the range of 40-60% by weight of the gum base.

The resin may comprise a natural resin and/or it may comprise a synthetic resin.

The resin may comprise a biodegradable resin. A biodegradable resin may e.g. be a polyester, e.g. obtained by polymerization of at least one cyclic ester.

In an embodiment of the invention, the gum base is a biodegradable gum base, and preferably a biodegradable gum base comprises at least one polyester polymer. A number of useful biodegradable polymers including biodegradable polyesters are found in PCT application no. WO 2003/DK/00941, which is incorporated herein by reference.

The gum base may furthermore comprise one or more components selected from the group consisting of an emulsifier, a fat, a wax, and filler.

In a preferred embodiment, the gum base comprises emulsifier in an amount in the range of 1-15% by weight, and preferably in the range 5-10% by weight.

A number of different emulsifiers may be used in the gum base. For example, anionic, cationic, amphoteric or non-ionic emulsifiers can be used. Suitable emulsifiers include lecithins, polyoxyethylene stearate, polyoxyethylene sorbitan fatty acid esters, fatty acid salts, mono and diacetyl tartaric acid esters of mono and diglycerides of edible fatty acids, citric acid esters of mono and diglycerides of edible fatty acids, saccharose esters of fatty acids, polyglycerol esters of fatty acids, polyglycerol esters ofinteresterified castor oil acid (E476), sodium stearoyllatylate, sodium lauryl sulfate and sorbitan esters of fatty acids and polyoxyethylated hydrogenated castor oil (e.g. the product sold under the trade name CREMOPHOR), block copolymers of ethylene oxide and propylene oxide (e.g. products sold under trade names PLURONIC and POLOXAMER), polyoxyethylene fatty alcohol ethers, polyoxyethylene sorbitan fatty acid esters, sorbitan esters of fatty acids and polyoxyethylene steraric acid esters.

Particularly suitable emulsifiers are polyoxyethylene stearates, such as for instance polyoxyethylene (8) stearate and polyoxyethylene (40) stearate, the polyoxyethylene sorbitan fatty acid esters sold under the trade name TWEEN, for instance TWEEN 20 (monolaurate), TWEEN 80 (monooleate), TWEEN 40 (monopalmitate), TWEEN 60 (monostearate) or TWEEN 65 (tristearate), mono and diacetyl tartaric acid esters of mono and diglycerides of edible fatty acids, citric acid esters of mono and diglycerides of edible fatty acids, sodium stearoyllactylate, sodium laurylsulfate, polyoxyethylated hydrogenated castor oil, blockcopolymers of ethylene oxide and propyleneoxide and polyoxyethylene fatty alcohol ether. The emulsifiers may either be a single compound or a combination of several compounds.

In an embodiment of the invention, the gum base comprises at least one resin in an amount in the range of 10-90% by weight of the gum base, at least one elastomer in an amount in the range of 4-60% by weight of the gum base, and an emulsifier in an amount in the range of 1-15% by weight. Preferably, the gum base comprises at least one resin in an amount in the range of 30-70% by weight of the gum base, at least one elastomer in an amount in the range of 5-40% by weight of the gum base, and an emulsifier in an amount in the range of 5-10% by weight.

The mixture comprises at least one chewing gum ingredient and typically several chewing gum ingredients.

The at least one chewing gum ingredient may be selected from the group consisting of a bulk sweetener, a high intensity sweetener, a flavouring agent, a cooling agent, a warming agent a softeners, an emulsifier, a colouring agent, a binding agent, an acidulant, a filler, and an antioxidant.

Useful cooling agents are mentioned in U.S. Patent No. 6,627,233, the contents of which are incorporated herein by reference for all purposes.

Particular examples of cooling agents include:
substituted p-menthanes, substituted p-menthane-carboxamides (e.g., N-ethyl-p-menthane-3-carboxamide (FEMA 3455)), acyclic carboxamides, substituted cyclohexanamides, substituted cyclohexane carboxamides, substituted ureas and sulphonamides, and substituted menthanols (all from Wilkinson Sword); hydroxymethyl and hydroxyethyl derivatives of p-menthane (from Lever Bros.); menthyl succinate; 2-mercapto-cyclo-decanone (from International Flavors and Fragrances); 2-isopropanyl-5-methylcyclohexanol (from Hisamitsu Pharmaceuticals, hereinafter "isopregol"); hydroxycarboxylic acids with 2-6 carbon atoms; menthone glycerol ketals (FEMA 3807, tradename FRESCOLAT(TM) type MGA); 3-1-menthoxypropane-1,2-diol (from Takasago, FEMA 3784, (hereinafter "TCA")); menthyl lactate; (from Haarman & Reimer, FEMA 3748, tradename FRESCOLAT(TM) type ML).

The inventor has discovered that chokeberry, when released via the chewing gum composition, is accompanied by an unpleasant, bitter taste. A consumer acceptable taste and mouth feel may be important parameters of a chewing gum and for some uses bitter tastes should be avoided or at least reduced. The inventor has found that adding polyol to the mixture surprisingly masks the bitter taste of chokeberry.

Useful warming agents include capsicum and nicotinate esters, such as benzyl nicotinate.

In a preferred embodiment of the invention, the at least one chewing gum ingredient is a bulk sweetener, and preferably a polyol.

A number of useful polyols are available and the polyol may e.g. be selected from the group consisting of dextrose, sucrose, lactose, hydrogenated starch hydrolysates, xylitol, mannitol, sorbitol, mannitol, maltitol, isomaltol, erythritol, lactitol, maltodextrin, and cyclodextrin.

In an especially preferred embodiment of the invention, the polyol is present in amount ranging from 10-80% by weight of the mixture. This embodiment has the distinct advantage that the bitter taste associates with chokeberry is efficiently masked.

The polyol may be present in amount ranging from 30-70% by weight of the mixture, such as e.g. in the range 35-65%, and in the range 40-60%.

For example, the polyol may be present in amount ranging from 20-55% by weight of the mixture, such as e.g. in amount ranging from 30-50% by weight of the mixture.

The mixture may furthermore comprise a high intensity sweetener. The high intensity sweetener may e.g. be selected from the group consisting of sucralose, neotame, aspartame, salts of acesulfame, alitame, saccharin and its salts, cyclamic acid and its salts, glycyrrhizin, dihydrochalcones, thaumatin, monellin, stevioside, and combinations thereof.

The high intensity sweetener may e.g. be present in an amount ranging from 0.01-3% by weight of the mixture.

The chewing gum composition, and e.g. the mixture, may furthermore comprise an active ingredient.

Examples of such active ingredients is found e.g. in WO 00/25598, the contents of which are incorporated herein by reference for all purposes.

Useful active ingredients include drugs, dietary supplements, antiseptic agents, pH-adjusting agents, anti-smoking agents and substances for the care or treatment of the oral cavity and the teeth and compounds capable of releasing urea during chewing.

Examples of active ingredients are phenols (e. g. thymol, p-chlorophenol, cresol, EGCG), hexachlorophene, salicylic anilide compounds, triclosan, halogenes (iodine, iodophores, chloroamine, dichlorocyanuric acid salts), alcohols (3,4 dichlorobenzyl alcohol, benzyl alcohol, phenoxyethanol, phenylethanol), cf. also Martindale, metal salts, complexes and compounds with limited water- solubility, such as aluminum salts, (for instance aluminum potassium sulphate AIK(SO4) 2,12H20) and salts, complexes and compounds of boron, barium, strontium, iron, calcium, zinc, (zinc acetate, zinc chloride, zinc gluconate), copper (copper chloride, copper sulphate), lead, silver, magnesium, sodium, potassium, lithium, molybdenum, vanadium should be included; other compositions for the care of mouth and teeth: for instance; salts, complexes and compounds containing fluorine (such as sodium fluoride, sodiummonofluorophosphate, aminofluorides, stannous fluoride), phosphates, carbonates and selenium, Acceptable bases, such as carbonates, hydrogen carbonates, phosphates, sulphates or oxides of sodium, potassium, ammonium, magnesium or calcium, especially magnesium and calcium. Further active ingredients can be found in J. Dent. Res. Vol. 28 No. 2, pages 160-171, 1949, the contents of which are incorporated herein by reference for all purposes.

Active ingredients may comprise the below-mentioned compounds or derivates thereof, but are not limited thereto: Acetylsalicylsyre Buprenorphine, Ibuprofen, Ketoprofen, Morphine, Naproxen, Oxycodon, Piroxicam, Pseudoefedrin, Calciumcarbonat, Nicotine, Difenhydramine, Chlorhexidine, Urea, Aspirin, Aluminum salts, Calcium salts, Ferro salts, Silver salts, Zinc-salts, Caffeine, Ephedrine, Phenylephedrine, Nitroglycerin, Testosterone, Protothenic Acid, Aluminiumaminoacetat, paracetamol, benzocaine, cinnarizine, menthol, carvone, chlorhexidine-di-acetate, cyclizine hydrochloride, miconazole, aspartame, sodium fluoride, saccharin, cetylpyridinium chloride, other quaternary ammoniumcompounds, glibenclamide, acetyl- salicylic acid, sodium hydrogencarbonate, the active components from ginkgo, the active components from propolis, the active components from ginseng, methadone, oil of peppermint, salicylamide, hydrocortisone or astemizole, complexes and compounds containing fluorine (such as sodium fluoride, sodiummono- fluorophosphate, aminofluorides, stannous fluoride), phosphates, carbonates and selenium.

Other useful active ingredients may comprise the below-mentioned compounds or derivates thereof, but are not limited thereto: salicylic acid, salicyl amide and related substances (acetylsalicylic acid, choline salicylate, magnesium salicylate, sodium salicylate), paracetamol, salts of pentazocine (pentazocine hydrochloride and pentazocinelactate), buprenorphine hydrochloride, codeine hydro- chloride and codeine phosphate, morphine and morphine salts (hydrochloride, sulfate, tartrate), methadone hydrochloride, ketobemidone and salts of ketobemidone (hydrochloride), beta-blockers, (propranolol), calcium antagonists, verapamil hydrochloride, nifedinpine as well as suitable substances and salts thereof mentioned in Pharm. Int. , Nov. 85, pages 267-271, Barney H. Hunter and Robert L. Talbert, nitroglycerine, erythrityltetranitrate, strychnine and salts thereof, lidocaine, tetracaine hydrochloride, etorphine hydrochloride, atropine, insulin, enzymes (for instance papain, trypsin, amyloglucosidase. glucoseoxidase, streptokinase, streptodornase, dextranase, alpha amylase), polypeptides (oxytocin, gonadorelin, (LH. RH), desmopressin acetate (DDAVP), isoxsuprine hydrochloride, ergotamine compounds, chloroquine (phosphate, sulfate), isosorbide, demoxytocin, heparin.

Examples of active ingredients in the form of dietary supplements are for instance salts and compounds having the nutritive effect of vitamin B2 (riboflavin), B12, Folic acid, niacin, biotin, amino acids, the vitamins A, B, C, D, E and K, minerals in the form of salts, complexes and compounds containing calcium, phosphorus, magnesium, iron, zinc, copper, iodine, manganese, chromium, selenium, molybdenum, potassium, sodium or cobalt.

Dental products include Carbamide, CPP Caseine Phospho Peptide; Chlorhexidine, Chlorhexidine di acetate, Chlorhexidine Chloride, Chlorhexidine di gluconate, Hexetedine, Strontium chloride, Potassium Chloride, Sodium bicarbonate, Sodium carbonate, containing ingredients, Fluorides, Sodium fluoride, Aluminum fluoride, Ammonium fluoride, Calcium fluoride, Stannous fluoride, Other fluor containing ingredients Ammonium fluorosilicate, Potasium fluorosilicate, Sodium fluorosilicate, Ammonium monofluorphosphate, Calcium monofluorphosphate, Potassium monofluorphosphate, Sodium monofluorphosphate, Octadecentyl Ammonium fluoride, Stearyl Trihydroxyethyl PropylenediamineDihydrofluoride, Vitamins include A, B1, B2, B6, B12, Folic acid, niacin, Pantothensyre, biotin, C, D, E, K. Minerals include Calcium, phosphor, magnesium, iron, Zink, Cupper, Iodine, Manganese, Chromium, Selenium, Molybdenum. Other active ingredients include: Q10, enzymes.

The active ingredient may also be one or more migraine drugs such as Serotonin antagonists: Sumatriptan, Zolmitriptan, Naratriptan, Rizatriptan, Eletriptan; nausea drugs such as Cyclizin, Cinnarizin,Dimenhydramin, Difenhydrinat; hay fever drugs such as Cetrizin, Loratidin, pain relief drugs such as Buprenorfin, Tramadol, oral disease drugs such as Miconazol, Amphotericin B, Triamcinolonaceton; and the drugs Cisaprid, Domperidon, Metoclopramid.

In an embodiment of the invention, the chewing gum composition comprises a first layer of compressed mixture.

The chewing gum composition may further comprise a second layer of a compressed composition substantially free of gum base.

The chewing gum composition may furthermore comprise a further layer of a compressed mixture.

In an embodiment of the invention, the chewing gum composition comprises a center filling. The mixture may e.g. comprise the center filling.

The mixture of the chewing gum composition may be processed into in a number of different shapes. The mixture may for example be shaped as a stick, a core, a tablet, a slab, a bead, a pellet, a tape, or a ball.

A chewing gum composition according to the present invention, has typically a weight in the range of 0.1-10 g, such as in the range of 0.5-5 g or in the range of 0.75-2.5 g, preferably in the range of 0.8-2 g, and even more preferred in the range of 1-1.5 g. Center filled chewing gum compositions normally have weights in the range of 0.5-5 g, preferably in the range of 1-4 g, and even more preferred in the range of 2-3 g. Typical weights for bead shaped chewing gum compositions are in the range of 0.1-0.6 g, preferably in the range of 0.2-0.5 g, and even more preferred in the range of 0.3-0.4 g.

An interesting effect of the chewing gum composition is its ability to retain a significant fraction of the chokeberry in the mixture, even after prolonged chewing. As demonstrated in the Example 2, about 20% percent of the chokeberry is still present in the mixture after 15 minutes chewing. This ability is particularly vital for the "chew out" indicator effect.

In an embodiment of the invention at most 95% by weight of the anthocyanin comprised in the chokeberry is released within the first 15 minutes of chewing the chewing gum composition.

Preferably at most 85% by weight of the anthocyanin comprised in chokeberry is released within the first 15 minutes of chewing the chewing gum composition. For example, about 80% by weight of the anthocyanin comprised in chokeberry may be released within the first 15 minutes of chewing.

It may be preferred that at most 95% by weight of the anthocyanin comprised in the chokeberry of the mixture is released within the first 15 minutes of chewing the chewing gum composition.

For example at most 85% by weight of the anthocyanin comprised in the chokeberry of the mixture is released within the first 15 minutes of chewing the chewing gum composition, such as about 80% by weight of the anthocyanin comprised in the chokeberry of the mixture may be released within the first 15 minutes of chewing.

Release of anthocyanin from the chewing gum composition, and preferably from the mixture, may be determined by means of the chewing machine and methodology described in the European Pharmacopoeia No. 4, 2.9.25.

In a preferred embodiment of the invention, the chewing gum composition comprises a mixture comprising
- a gum base in an amount of 30-50% by weight the mixture,
- polyol in an amount of 40-60% by weight the mixture,
- high intensity sweetener in an amount of 0.1-0.6% by weight the mixture,
- chokeberry in an amount of 0.5-5% by weight of the mixture, and where chokeberry comprises anthocyanin in an amount ranging from 15-50% by weight.

Another aspect of the invention relates to a granule of the mixture as described herein. In an embodiment of the invention, the granule comprise the mixture comprising at least one chewing gum ingredient, gum base, and chokeberry, the mixture comprising gum base in an amount of 10-70% by weight.

The granule may e.g. be prepared by a method comprising the steps of mixing the at least one chewing gum ingredient, the gum base, and chokeberry, and forming one or more granules from the resulting mixture.

In an embodiment of the invention, the size of the granule is in the range of 0.01-2 mm, preferably within the range of 0.1 mm- 1 mm.

The granules are e.g. useful for preparing compressed chewing gum compositions, i.e. chewing gum compositions with one or more compressed layers.

In an alternative aspect of the invention it is envisioned that chokeberry as mentioned herein can be replaced by anthocyanin, i.e. monomeric anthocyanin molecules and/or proanthocyanin molecules.

In yet an alternative aspect of the invention, it is envisioned that chokeberry as mentioned herein can be replaced by encapsulated anthocyanin. The encapsulated anthocyanin may e.g. be prepared using the encapsulation methods and encapsulation materials described herein.

A further aspect of the invention relates to a method of preparing the chewing gum composition according to the present invention. The method comprises the steps of mixing the at least one chewing gum ingredient, the gum base, and chokeberry; shaping the mix to obtain the mixture; and, optionally, coating the mixture.

The method may be performed as a batch process. Alternatively it may be performed as a continuous process.

It is preferred that the temperature of the gum base is at most 70°C, and even more preferred at most 65°C, when chokeberry is mixed with the gum base.

In an embodiment of the invention, the chokeberry is applied to the mixture after the gum base has been mixed with the at least one chewing gum ingredient, e.g. a bulk sweetener such as a polyol.

Anthocyanins are known to be potent antioxidants and the chewing gum composition of the invention can therefore advantageously be used as a source of antioxidant. Thus, another aspect of the invention relates to the use of the chewing gum composition as described herein as a source of antioxidants.

This use corresponds to a method of using of the chewing gum composition as described herein as a source of antioxidants by orally administering the chewing gum composition to an individual.

An additional aspect relates to use of chokeberry as a pH indicator in a chewing gum composition as defined herein and particularly as a pH indicator in the mixture of a chewing gum composition.

As mentioned, the inventor has discovered a surprising additional effect of the chewing gum composition, namely its ability to change colour after 3-5 minutes of chewing. Without being bound by theory it is believed that chokeberry inherently is acidic and slowly is titrated by the neutral saliva. The colour change, which is observed after prolonged chewing, is useful to signal to the consumer that now the chewing gum has been sufficiently chewing. This is particularly important and useful in the case of chewing gum compositions containing active ingredient such as pharmaceuticals and nutraceuticals. The active ingredient must normally be release in a certain dose to be effective, and the colour change of the chewing gum composition may signal that active ingredient now has been sufficiently released/"chewed out". In the present context this effect is called "chew out" indication.

Thus, an aspect relates to the use of chokeberry as a "chew out" indicator in a chewing gum composition as defined herein, and particularly as a "chew out" indicator in the mixture of a chewing gum composition.

As mentioned chokeberry is believed to inhibit the colonization of streptococci in the initial phase of biofilm formation hence slow the development of dental plaque.

Thus, another aspect of the present invention relates to the use the chewing gum composition as defined herein for cosmetic or non-cosmetic prevention and/or removal of plaque at the dental surface.

This use corresponds to a method of using of the chewing gum composition as described herein for cosmetic or non-cosmetic prevention and/or removal of plaque at the dental surface by orally administering the chewing gum composition to an individual.

Particularly, the high molecular weight of proanthocyanins from chokeberry is believed to inhibit the biofilm formation of oral Streptococcus.

Thus, a further aspect of the present invention relates to the use of the chewing gum composition as defined herein for cosmetic or non-cosmetic prevention and/or removal of biofilm at the dental surface.

This use corresponds to a method of using of the chewing gum composition as described herein for cosmetic or non-cosmetic prevention and/or removal of biofilm at the dental surface by orally administering the chewing gum composition to an individual.

The controlled, delayed release of the chokeberry and anthocyanin, which is observed from the present chewing gum composition is believed to be especially advantageous for prevention and/or removal of biofilms and for the prevention and/or removal of plaque.

The individual as mentioned herein may be a mammal, and preferably a human.

The above-mentioned uses and methods may also be used for curative or prophylactic purpose.

It should be noted that, according to the present invention, embodiments and features described in the context of one of the aspects of the present invention also apply to the other aspects of the invention.

### EXAMPLES

### Example 1 Chewing gum compositions containing chokeberry

A number of specific chewing gum composition comprising chokeberry have been listed in Table 1.

Chewing gum composition #3 was prepared using a gum base containing about 15% by weight elastomer, about 20% by weight natural resin, about 20% by weight PVA, about 20% by weight filler, about 5% emulsifier, and about 20% by weight fat.

The gum base was ground with some of the sorbitol powder in a Z-arm mixer at a temperature in the range of 60-65°C. Subsequently the remaining components were added to and mixed into the mixture. The mixture was then shaped in to cores of chewing gum with an average weight of about 1 g. The chokeberry juice was an up-concentrated juice having an anthocyanin content in the range of 10-20% by weight, and having an intensely bitter taste.

**Table 1 Components of the chewing gum compositions**

| **Component** | **Amount (g)** | | | | | |
|---|---|---|---|---|---|---|
| **#** | **1** | **2** | **3** | **4** | **5** | **6** |
| Gum base | 50 | 110 | 160 | 210 | 260 | 310 |
| Sorbitol Powder | 310 | 250 | 200 | 150 | 100 | 50 |
| Maltitol syrup | 12 | 12 | 12 | 12 | 12 | 12 |
| Acesulfame | 0.8 | 0.8 | 0.8 | 0.8 | 0.8 | 0.8 |
| Aspartame | 0.8 | 0.8 | 0.8 | 0.8 | 0.8 | 08 |
| Chokeberry juice | 4.8 | 4.8 | 4.8 | 4.8 | 4.8 | 4.8 |
| Flavour | 14 | 14 | 14 | 14 | 14 | 14 |

### Example 2 Testing the release of anthocyanin from the chewing gum composition in vitro

Anthocyanin is one of the major components of chokeberry and is a useful marker for measuring release of chokeberry. The release of anthocyanin from the chewing gum #3 was tested by a time-release experiment by in vitro chewing using a chewing machine according to the European Pharmacopoeia No. 4, 2.9.25. The release was measured indirectly by determining the anthocyanin remaining in the chewed chewing gum composition.

The determination was performed by means of High Pressure Liquid Chromatography (HPLC) and an ultraviolet photodiode array (UV-PDA) for measuring absorbance at 520 nm.

The result is plotted in Figure 1 and shows a controlled, delayed release of the anthocyanin. About 80% of the anthocyanin was released during the first 15 minutes of chewing the chewing gum and the remaining 20% were still in the mixture.

### Example 3 In vivo test of the chewing gum composition

Four test persons tried chewing chewing gum composition #3. It was noted that the bitterness of chokeberry was efficiently masked in the chewing gum composition. Furthermore it was noted that the chewing gum composition changed colour from intensely red to blue/purple after about 3-5 minutes of chewing.

## Claims

1. A chewing gum composition comprising a mixture, said mixture comprising at least one chewing gum ingredient, gum base, and chokeberry, the mixture comprising gum base in an amount of 10-70% by weight.

2. The chewing gum composition according to claim 1, the chewing gum composition further comprising a coating.

3. The chewing gum composition according to claim 2, wherein the coating further comprises chokeberry.

4. The chewing gum composition according to any of the preceding claims, further comprising a high intensity sweetener.

5. The chewing gum composition according to any of the preceding claims, wherein at most 90% by weight of the anthocyanin comprised in said chokeberry is released within the first 15 minutes of chewing.

6. The chewing gum composition according to any of the preceding claims, wherein said chokeberry is a powder of the dry-matter of a chokeberry juice or a chokeberry extract.

7. The chewing gum composition according to any of the preceding claims, wherein said chokeberry is encapsulated chokeberry.

8. The chewing gum composition according to claim 7, wherein the encapsulated chokeberry is an encapsulated chokeberry granulate.

9. A granule comprising a mixture, said mixture comprising at least one chewing gum ingredient, gum base, and chokeberry, the mixture comprising gum base in an amount of 10-70% by weight.

10. A method of preparing a chewing gum composition according to any of claims 1-8, comprising the steps of mixing the at least one chewing gum ingredient, the gum base, and chokeberry; shaping the mix to obtain the mixture; and, optionally, coating the mixture.

11. Use of the chewing gum composition according to any of claims 1-8 as a source of antioxidants.

12. Use of the chewing gum composition according to any of claims 1-8 as a pH indicator.

13. Use of the chewing gum composition according to any of claims 1-8 as a "chew out" indicator.

14. Use of a chewing gum composition according to any of claims 1-8 for cosmetic prevention and/or removal of plaque at the dental surface.

15. Use of a chewing gum composition according to any of claims 1-8 for cosmetic prevention and/or removal of biofilm at the dental surface.

## Patentansprüche

1. Kaugummizusammensetzung, umfassend eine Mischung, wobei die Mischung mindestens einen Kaugummibestandteil, Kaumasse und Aronia umfasst, wobei die Mischung Kaumasse in einer Menge von 10-70 Gew.-% umfasst.

2. Kaugummizusammensetzung nach Anspruch 1, wobei die Kaugummizusammensetzung weiterhin einen Überzug umfasst.

3. Kaugummizusammensetzung nach Anspruch 2, wobei der Überzug weiterhin Aronia umfasst.

4. Kaugummizusammensetzung nach einem der vorhergehenden Ansprüche, weiterhin umfassend einen Süßstoff mit hoher Süßkraft.

5. Kaugummizusammensetzung nach einem der vorhergehenden Ansprüche, wobei höchstens 90 Gew.-% des in Aronia enthaltenen Anthocyanins innerhalb der ersten 15 Kauminuten freigesetzt werden.

6. Kaugummizusammensetzung nach einem der vorhergehenden Ansprüche, wobei Aronia ein Pulver der Trockenmasse von Aronia-Saft oder Aronia-Extrakt ist.

7. Kaugummizusammensetzung nach einem der vorhergehenden Ansprüche, wobei Aronia verkapseltes Aronia ist.

8. Kaugummizusammensetzung nach Anspruch 7, wobei das verkapselte Aronia ein verkapseltes Aronia-Granulat ist.

9. Granulatkorn, umfassend eine Mischung, wobei die Mischung mindestens einen Kaugummibestandteil, Kaumasse und Aronia umfasst, wobei die Mischung Kaumasse in einer Menge von 10-70 Gew.-% umfasst.

10. Verfahren zur Herstellung einer Kaugummizusammensetzung nach einem der Ansprüche 1-8, umfassend die Schritte des Mischens von mindestens einem Kaugummibestandteil, Kaumasse und Aronia; Formen der Mischung zum Erhalt der Mischung und fakultativ Beschichten der Mischung.

11. Verwendung der Kaugummizusammensetzung nach einem der Ansprüche 1-8 als Quelle für Antioxidanzien.

12. Verwendung der Kaugummizusammensetzung nach einem der Ansprüche 1-8 als pH-Indikator.

13. Verwendung der Kaugummizusammensetzung nach einem der Ansprüche 1-8 als Indikator dafür, dass der Kaugummi "ausgekaut" ist.

14. Verwendung einer Kaugummizusammensetzung nach einem der Ansprüche 1-8 für die kosmetische Vorbeugung und/oder das Entfernen von Plaque auf Zahnoberflächen.

15. Verwendung einer Kaugummizusammensetzung nach einem der Ansprüche 1-8 für die kosmetische Vorbeugung und/oder das Entfernen von biologischen Filmen auf Zahnoberflächen.

## Revendications

1. Composition de gomme à mâcher comprenant un mélange, ledit mélange comprenant au moins un ingrédient pour gomme à mâcher, une base de gomme et de l'aronie, le mélange comprenant 10 à 70 % en poids de base de gomme.

2. La composition de gomme à mâcher selon la revendication 1, la composition de gomme à mâcher comprenant également un enrobage.

3. La composition de gomme à mâcher selon la revendication 2, dans laquelle l'enrobage comprend également de l'aronie.

4. La composition de gomme à mâcher selon l'une quelconque des revendications précédentes, comprenant également un édulcorant intense.

5. La composition de gomme à mâcher selon l'une quelconque des revendications précédentes, dans laquelle au plus 90 % en poids de l'anthocyane comprise dans ladite aronie est libérée pendant les 15 premières minutes de mastication.

6. La composition de gomme à mâcher selon l'une quelconque des revendications précédentes, dans laquelle ladite aronie est une poudre de matière sèche d'un jus d'aronie ou d'un extrait d'aronie.

7. La composition de gomme à mâcher selon l'une quelconque des revendications précédentes, dans laquelle ladite aronie est de l'aronie encapsulée.

8. La composition de gomme à mâcher selon la revendication 7, dans laquelle l'aronie encapsulée est un granulé d'aronie encapsulé.

9. Granulé comprenant un mélange, ledit mélange comprenant au moins un ingrédient pour gomme à mâcher, une base de gomme et de l'aronie, le mélange comprenant 10 à 70 % en poids de base de gomme.

10. Méthode de préparation d'une composition de gomme à mâcher selon l'une quelconque des revendications 1 à 8, comprenant les étapes de mélange de l'au moins un ingrédient pour gomme à mâcher, de la base de gomme et de l'aronie ; de mise en forme de ce mélange pour obtenir le mélange ; et, éventuellement, d'enrobage du mélange.

11. Utilisation de la composition de gomme à mâcher selon l'une quelconque des revendications 1 à 8 comme source d'antioxydants.

12. Utilisation de la composition de gomme à mâcher selon l'une quelconque des revendications 1 à 8 comme indicateur de pH.

13. Utilisation de la composition de gomme à mâcher selon l'une quelconque des revendications 1 à 8 comme indicateur de « fin de mastication ».

14. Utilisation d'une composition de gomme à mâcher selon l'une quelconque des revendications 1 à 8 à des fins de prévention cosmétique et/ou d'élimination de la plaque dentaire de la surface des dents.

15. Utilisation d'une composition de gomme à mâcher selon l'une quelconque des revendications 1 à 8 à des fins de prévention cosmétique et/ou d'élimination du biofilm de la surface des dents.
